# EUROPEAN PATENT SPECIFICATION

(11) **EP 0 217 917 B1**
(45) Date of publication and mention of the grant of the patent: **15.12.1993**
(21) Application number: 86902601.3
(22) Date of filing: 26.03.1986
(51) Int. Cl.: C12P 21/00, C12P 1/04, C12P 1/02, C12N 15/00, C12N 11/14, C12N 11/02, C12N 11/08, C12P 5/00, C07K 15/20

(54) **WEIGHTED MICROSPONGE FOR IMMOBILIZING BIOACTIVE MATERIAL**
GEFÜLLTER MIKROSCHWAMM ZUR IMMOBILISIERUNG BIOAKTIVER MATERIALIEN
MICROEPONGES CHARGEES POUR IMMOBILISER DES SUBSTANCES BIOACTIVES

(30) Priority: 04.04.1985 US 719880; 04.04.1985 US 719881; 10.05.1985 US 732736
(43) Date of publication of application: 15.04.1987
(73) Proprietor: VERAX CORPORATION, Hanover, NH 03755 (US)
(72) Inventor: DEAN, Robert, C., Norwich, VT 05055 (US); PHILLIPS, Philip, G., Norwich, VT 05055 (US); RUNSTADLER, Peter, W., Jr., Hanover, NH 03755 (US); SILVER, Frederick, H., Long Valley, NJ 07853 (US); BERG, Richard, A., Lambertville, NJ 08530 (US); CAHN, Frederick, Belmont, MA 02178 (US)
(74) Representative: Dean, John Paul
(86) International application number: US8600600
(87) International publication number: WO8605811

(56) References cited:
- EP-A- 0 053 869
- EP-A- 0 097 907
- EP-A- 0 109 124
- WO-A-82/00660
- FR-A- 2 464 994
- US-A- 3 242 055
- US-A- 3 977 941
- US-A- 4 090 022
- US-A- 4 247 498
- US-A- 4 286 061
- US-A- 4 373 027
- US-A- 4 412 947
- US-A- 4 430 760
- Lindsey et al., FEBS Letters 155(1): 143-149 (1983)
- Biopolymers, vol. 4, issued 1966, I.H. Silman et al., "Some Water-Insoluble Papain Derivtives", pp. 444-445, s. paragraph bridging

## Description

This invention was made in the course of, or under, a contract with NIH. The government has rights to the invention pursuant to SBIR Grant No. CA37430.

### BACKGROUND OF THE INVENTION

### Field of the Invention

The present invention pertains to the art of immobilizing bioactive materials and particularly relates to an improved microsponge for use in motive bioreactor systems. The present invention also relates to the art of culturing microorganisms and cells, hereinafter referred to collectively as organisms, and particularly relates to the culturing of organisms immobilized on and/or in microsponges in motive reactor systems as submerged suspensions.

### Description of the Prior Art

A large variety of chemical products are produced by culturing organisms. For example, fermentation processes abound for producing antibiotics and other drugs, alcoholic beverages, cheeses and the like. Most fermentation processes, however, are carried out commercially using batch reaction procedures, even though the value of continuous culturing techniques are recognized.

Recent advances in the areas of genetic engineering and medicine have ushered in a new era in the production of chemical products by culturing organisms. For example, hybridized mammalian cells or hybridomas have been developed which secrete antibodies specific to known antigens, such as tetanus toxoid. Bacteria, such as E. coli, have been genetically engineered to produce specific proteins, such as insulin. To best utilize this new technology, however, techniques superior to the batch fermentation processes must be developed for continuously culturing such organisms at high concentration and under conditions of optimal growth so as to maximize the production of such products.

Various arrangements for immobilizing bioactive materials are known. Solid supports have long been used for immobilizing microorganisms in the treatment of waste water and related fermentation processes. More recently, solid microcarriers have been used to obtain high cell densities in the culture of attachment-dependent cells. For example, microporous polymeric supports fabricated for example from dextran have been used for cultivating cells. Such supports can be obtained commercially from Pharmacia Fine Chemicals under the brand name Cytodex®. Such solid bio-supports, however, are not suitable for motive reactor systems such as vigorously stirred tanks and fluidized beds since substantially all of the cells are adherent to the surface of said supports and thus are exposed to impact stress and trauma during operation.

Porous inorganic microcarriers also are known and such supports potentially provide protection for the cells in motive applications since the cells populate the interior of the microcarriers. Unfortunately, inorganic microcarriers cannot be made with the proper combination of permeability and specific gravity to function well in all motive applications. For example, the porous fritted glass or cordierite supports described in Messing et al. U.S. 4,153,510 would typically exhibit specific gravities in aqueous suspension of less than about 1.3 if their void fractions are greater than about 80% (Note that void fractions for the Messing supports are not disclosed). Quite understandably, these supports are not suitable for all motive reactor systems where a higher specific gravity generally is needed to ensure high relative velocities for maximum rates of mass and energy transfer. Consequently, these supports have generally been relegated for use in packed bed applications.

Additionally, it is not known how suitable these prior art microcarriers are for the cultivation of attachment-independent organisms such as hybridomas.

An object of the present invention is to provide a microsponge containing immobilized bioactive materials suitable for use in motive reactor systems.

Another object of the present invention is to provide a microsponge suitable for immobilizing a large variety of organisms characterized by wide variations in size and their degree of attachment to solid supports.

A further object of the present invention is to provide a microsponge suitable for motive reactor systems which permits the continued growth and reproduction of immobilized organisms.

It also is an object of the present invention to provide a microsponge suitable for motive reactor systems which is conducive to maximizing the metabolic activity of immobilized organisms.

Yet another object of the present invention is to provide a method for continuously culturing organisms at high concentrations.

Still another object of the present invention is to provide a microsponge suitable for motive reactor systems which permits the culturing of organisms at high concentrations while accommodating either maximum growth rate or maximum metabolic activity.

Another object of the present invention is to provide a method for the in vitro culturing of both attachment-dependent and attachment-independent organisms.

These and other objects of this invention will become apparent from a consideration of the specification and appended claims.

### SUMMARY OF THE INVENTION

The present invention pertains to a weighted microsponge, (preferably a collagen microsponge) for immobilizing bioactive materials in motive bioreactor systems, said microsponge comprising a porous, biostable, highly crosslinked collagen matrix containing an inert weighting material, said collagen matrix having an open to the surface pore structure with an average pore size in the range of from about 1 micron to about 150 microns, with the pores of said matrix occupying from about 70 to about 98% by volume of the microsponge, said microsponge also having an average particle size of from about 100 microns to about 1000 microns and a specific gravity of above about 1.05

The present invention also relates to a method for the continuous in vitro culture of organisms for the production of biochemical products comprising the steps of:
(a) providing a highly crosslinked, collagen microsponge prepared by
   (i) milling a source of collagen selected from the group consisting of Types I, II and III collagen,
   (ii) admixing said milled collagen with an acidic liquid medium,
   (iii) lyophilizing said collagen-liquid medium mixture into a dry sponge matrix, and
   (iv) crosslinking the collagen in said sponge matrix by a treatment selected from the group consisting of:
      (1) contacting said collagen with a crosslinking agent selected from the group consisting of carbodiimides and bifunctional succinimidyl active esters,
      (2) subjecting said dry sponge matrix to elevated temperatures under a vacuum, and
      (3) a combination thereof;
(b) inoculating the highly crosslinked, collagen microsponge of step (a) with a culture of said organisms;
(c) passing a nutrient medium in direct contact with the inoculated, highly crosslinked, collagen microsponge; and
(d) recovering said biochemical products with the nutrient medium effluent.

### BRIEF DESCRIPTION OF THE DRAWINGS

Figure 1 is a photomicrograph showing a suitable collagen microsponge matrix of the present invention illustrating a fibrous structure.

Figure 2 is a photomicrograph of another collagen microsponge matrix according to the present invention illustrating a leafy structure.

### DETAILED DESCRIPTION

The present invention is directed to a weighted microsponge, preferably a collagen microsponge, containing immobilized bioactive materials, particularly organisms, suitable for use in motive bioreactor systems and to a method for the continuous in vitro culture of organisms for the production of biochemical products. As used throughout the specification and claims, the term "bioactive material" broadly encompasses both enzymes and other chemical factors such as chelating agents, hormones, antibodies, etc., and organisms, i.e., microorganisms and the cells of higher organisms. As used throughout the specification and claims, the term "organism" broadly encompasses both mircoorganisms and the cells of higher organisms. The organisms may be derived without limitation from such diverse sources as bacteria, fungi, viruses, algae, yeasts, animal cells (tissue), e.g., mammals, insects and fish and plant cells. The term "organisms" and "cell" will be used interchangeably throughout the specification and claims. As also used throughout the specification and claims, the term "biochemical product" refers not only to both primary and secondary metabolites produced by such organisms, but also to the cellular material or biomass of the organism itself, containing, for example, non-secretory products. Since the weighted microsponge of the present invention has particular advantages when used for culturing organisms, it generally will be described with reference to such embodiments, although it is not to be so-limited.

The microsponge of this invention is prepared from a biocompatible (e.g., non-toxic) polymer that is stable in service for an appropriate period of time, e.g., on the order of months. The preferred microsponge of this invention is formed of a highly crosslinked collagen. Biocompatibility refers to the ability of the polymeric (e.g. collagen matrix material to support a viable culture of organisms without substantially adversely affecting any desired characteristic of the immobilized organisms, e.g., in the case of hybridomas, the polymeric (e.g. collagen matrix material must not reduce the production of monoclonal antibodies. The stability or biostability of the matrix material refers to its ability to maintain its strength and integrity under in vitro conditions over the relevant time period for culturing the organism of interest. For example, in the case of a hybridoma culture for producing monoclonal antibodies, it is expected that the motive bioreactor would be operated continuously for three to six months or more. Thus, the matrix material should be biostable for this time period.

Both natural and synthetic polymeric materials can be used as the matrix material. Examples of suitable polymers include polysaccharides such as dextran, dextrin, starch, cellulose, agarose, carrageenan and the like; proteins such as collagen and the like; and synthetic polymers such as polyvinyl alcohols, polyacrylates, polymethacrylates, polyacrylamides, polyesters, polyurethanes, polyamides and the like. Generally, a material's biocompatability and biostability can be verified using routine experimentation.

Based on its biocompatibility and strength, collagen is presently the material of choice. Collagen is a biodegradable polymer found in animals, including man. It has numerous uses in the medical art and in most applications is reconstituted and crosslinked into an insoluble form using various crosslinking agents, such as aldehydes, e.g., glutaraldehyde and formaldehyde; ethylchloroformate; dimethyl adipimidate; N,N-methylenebisacrylamide; 1,2-diacrylamide ethyleneglycol; cyanamide; N-N'-diallyltartardiamide; cyanogen bromide; concanavalin A; 6-aminohexanoic acid; 1,6-diaminohexane; succinimidyl active esters; carbodiimides and compounds having similar crosslinking groups and/or physical treatment techniques such as freeze-drying and severe dehydration at a vacuum of about 50 millitors or more and at a temperature ranging from 50°C to 200°C. Unfortunately, many if not all of the commonly used crosslinking agents, particularly glutaraldehyde, which are unavoidably present in the crosslinked collagen, cause adverse biological effects and so are cytotoxic.

Recently, a new collagen-based matrix of improved biostability has been discovered. This collagen material is prepared without the conventional crosslinking agents. In this matrix, Type I, II or III collagen is crosslinked using a carbodiimide or a succinimidyl active ester and/or severe dehydration conditions at temperatures ranging from 50° to 200°C. Such crosslinked collagen typically has a molecular weight of from about 1 x 10⁶ to in excess of 50 x 10⁶. The molecular weight of the collagen between adjoining crosslinks varies from about 1000 to 100,000 via the formation of covalent bonds. Because of its resistance to degradation by collagenase and other proteinases, this crosslinked collagen has been found to be particularly suitable as the porous matrix of the microsponge. In fact, when used for the continuous culture of immobilized organisms, particularly hybridoma cells expressing monoclonal antibodies, microsponges made from this collagen material have exhibited some surprising properties. For example, hybridomas cultured with essentially protein-free medium are much more effective in producing monoclonal antibodies when immobilized on and/or in such collagen matrix microsponges than when such matrix is not present. Furthermore, microsponges made from the noted crosslinked collagen material appear to preferentially retain high concentrations of living (viable) cells and expel non-viable cells.

The preferred crosslinked collagen can be prepared from both soluble collagens and insoluble collagens of the Types I, II and III. The soluble collagens are prepared by limited enzymatic digestion and/or extraction of tissue enriched in such collagen types. Insoluble collagens are derived from the following typical sources: Type I collagen: bovine, porcine, chicken and fish skin, bovine and chicken tendon and bovine and chicken bones including fetal tissues; Type II collagen: bovine articular cartilage, nasal septum, sternal cartilage; and Type III collagen: bovine and human aorta and skin. For example, Type I collagen from bovine corium may be used. It is preferred to use Type I tendon collagen.

In the broad practice of the present invention, the physical form or macro-configuration of the crosslinked collagen microsponge is not critical. The microsponge can be used in various physical shapes including beads, flakes, discs, fibers, films, coatings, e.g., on extended surface supports, and sheets. For example, the microsponge can be provided as a coating on such well-known supports as pellets, stars, spiral rings, cross partition rings, Raschig rings, Berl saddles, Intalox saddles, pall rings, Tellerette rings and the like. The microsponge also can be provided in sheet form, in tubular form or in any other unsupported structure.

In the broad practice of the process of the present invention, the size of the microsponge is not critical. For example, for packed bed or fixed bed applications (e.g., plug flow reactors) the microsponge may be provided in a size typical for such applications. In order to be suitable for culturing high concentrations of organisms in motive reactor systems and allow for the transfer of nutrients to the immobilized organisms and the transfer of desired products from the microsponge, the weighted microsponge, preferably collagen, of the present invention must satisfy several functional requirements. The microsponge typically is in the shape of a bead and should have a particle size within the range of about 100 microns to 1000 microns, preferably from about 200 microns to 500 microns. At larger particle sizes the entire internal volume of the porous structure is not utilized effectively for producing the desired product by reaction between the immobilized bioactive material and the liquid medium contacted therewith, thus degrading the volumetric productivity of the motive reactor employing such microsponges. Smaller particle sizes present practical problems in preparing the microsponge and in operating the motive reactor.

Permeability of the microsponge is another important consideration. A microsponge's permeability is determined by the interrelationship of its porosity or void fraction and its pore structure. Void fraction is defined as the ratio of the volume of interstices of a material to the total volume occupied by the material and often is expressed as a percentage. In order to permit operation at high organism concentrations, the microsponge should have a void fraction of between about 70% and 98%. Preferably the void fraction of the collagen microsponge is greater than 85% and most desirably is greater than about 90%.

The microsponge also must possess an open to the surface pore structure. This allows for cell entry, without excessive shear forces, cell retention, subsequent cell growth, and expulsion of excess cell mass. For example in cases where the desired product is not secreted by the organisms, e.g., genetically engineered E. coli with a non-expressed rDNA product such as insulin, the organisms must be able to escape the microsponge as the immobilized colony expands by division. An open pore structure is essential if this process is to proceed on a continuous basis, without rupturing the microsponge structure. The desired organism product is recovered as an entrained component of the culture harvest liquor.

The microsponge should contain pores with an average size within the range of about 1 micron for the smallest microbes and for viruses, up to about 150 microns for large mammalian and plant cells. Generally, the pores of the microsponge must be at least as large as the smallest major dimension of the immobilized bioactive material but less than about 5 times the largest major dimension. Preferably, the pore size of the matrix is on the order of 1.5 to 3 times the average diameter of the organism or cell. If unknown, the smallest and largest major dimensions of an organism can be determined using known techniques. Applicants have found that the recited combination of particle sizes and pore sizes insure adequate mass transfer of constituents such as nutrients to the immobilized organisms, as well as adequate mass transfer of constituents, such as desired metabolites from the immobilized organisms.

For use in motive reactor systems, the microsponge (e.g. collagen) also must be weighted. The polymeric materials (e.g. crosslinked collagen) used as the matrix material in the present invention generally have a specific gravity of about 1.0 or less. For proper operation in a motive reactor, a specific gravity of above about 1.05, preferably above about 1.3 and most preferably between about 1.6 and 2.0 is desired. It has been found surprisingly that it is possible to obtain collagen microsponges of the proper specific gravity by introducing certain weighting additives into the microsponge without undesirably reducing its void fraction. The weighting additive must be substantially inert in the reactor environment and non-toxic to the immobilized organism, or must be suitably treated to render the additive non-toxic. Also, the weighting additive should not adversely affect the productivity of the immobilized organism. Generally, materials, such as metals and their alloys and oxides, and ceramics, having a specific gravity above about 4 and preferably above about 7 are used. Examples of suitable weighting additives for use in the broad practice of this invention are chromium, tungsten, molybdenum, cobalt, nickel, titanium and their alloys, e.g., Monel, 316 stainless, Vitalium (a cobalt alloy with chromium and molybdenum), titanium 6Al-4V (a titanium alloy with aluminum and vanadium) and Haynes Stellite Alloy 25 (a cobalt alloy with chromium nickel, tungsten and manganese). Many of these materials, however, may not be compatible with certain organisms and routine experimentation will be necessary to assess toxicity for any application. For example, titanium is the weighting material of choice with hybridomas, since most other metals are cytotoxic.

The weighting additive can be introduced into and dispersed throughout the microsponge as a finely divided powder, with most particles having a size on the order of 10 to 40 microns. However, to minimize the surface area of the weighting additive, it is desirable to employ it as a solid core in the microsponge. Sufficient weighting material is added to yield a microsponge with the desired specific gravity. For example, about a 50 micron diameter core of a weighting additive having a specific gravity of about 7.0 coated with a 50 micron thick layer of collagen having an average pore size of 20 to 40 microns and a void fraction of about 99% yields a microsponge with a specific gravity of about 1.7 having an overall void fraction of about 85%. Such a microsponge is particularly suitable for use in an aerobic motive reactor systems.

Finally, for motive applications the collagen microsponge should exhibit the proper resistance to attrition. A charge of microsponges preferably should have a useful life on the order of three to six months or more. Typically, the microsponges should exhibit not greater than about a 10% loss in volume after three months of operation.

Normally, organisms exhibit wide variation in their degree of attachment to solid supports. Certain organisms, for example, readily cling or attach to a wide variety of supports, including both organic and inorganic materials, while others will only attach to supports of biological origin (attachment-dependent organisms). Other organisms exhibit little direct attachment to any support material (attachment-independent organisms). The microsponge of the present invention, because it is prepared from polymeric (organic) materials and because of its permeability (porosity and pore structure) should be suitable for immobilizing substantially all types of organisms.

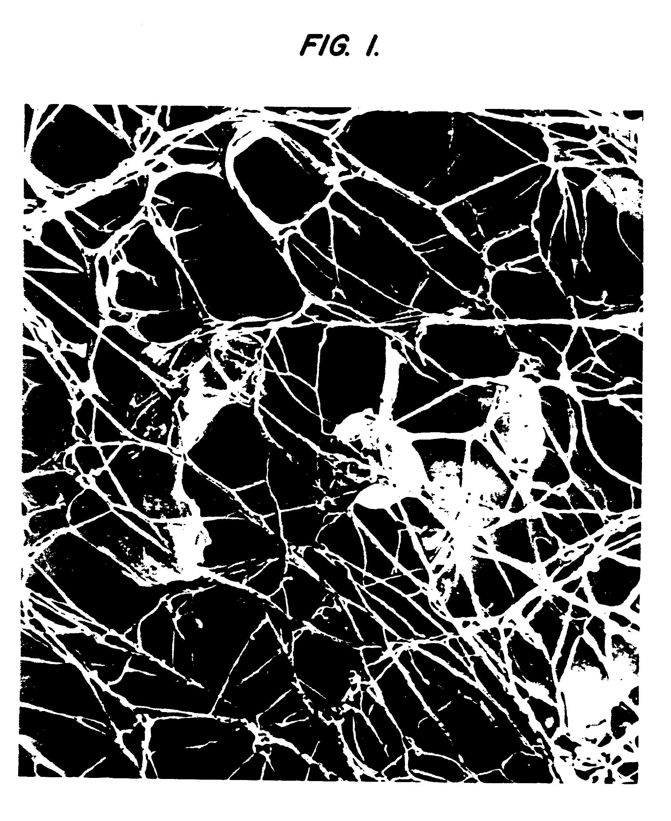

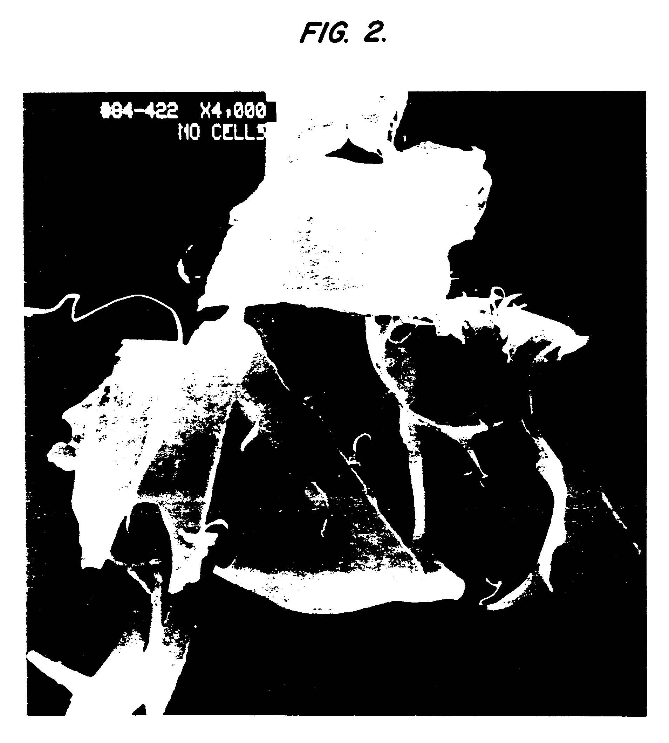

In fact, as noted in more detail below, it even is possible to tailor the micro-structure or configuration of the microsponge to best accommodate the attachment tendency of the immobilized organism. For example, microsponges having the wire-mesh structure (Figure 1) can be employed in conjunction with attachment-independent organisms, while microsponges exhibiting a leafy structure (Figure 2) can be used with attachment dependent organisms.

Any suitable procedure used by the prior art for immobilizing such organisms on microsponges can be used in the present invention including such techniques as adsorption and chemical coupling. For example, in the case of certain organisms it will only be necessary to mix the collagen microsponges in a broth inoculated with the specific organism. After a short period of time, the organism will colonize the microsponges and become entrapped in their pores. In the case of some organisms such as fibroblasts and hybridomas, it also may be desirable to coat the microsponge with attachment-promoting materials such as fibronectin, polylysine and anti-hybridoma antibodies prior to inoculation. Other techniques, such as applying a net charge to the surface of the microsponge, also can be used to enhance immobilization.

As will be recognized by those skilled in this art, in the broad practice of the present invention, the procedure used for bringing the immobilized bioactive material into direct contact with a liquid reagent stream such as a growth supporting medium for culturing of immobilized organisms is not critical and any of the numerous arrangements available in the prior art including such well known apparatus as stirred tank reactors, fixed bed reactors, fluidized bed reactors and moving bed reactors and the like could be used. Generally, when culturing organisms the microsponges are charged to a suitable reactor and mixed therein with a nutrient broth and an inoculum of the organism. The microsponges should be completely submerged. The microsponges are incubated so that the organisms grow and colonize the porous matrix of the microsponge. Fresh nutrient broth along with other materials necessary for growth, such as oxygen in the case of aerobic organisms, are supplied in a continuous manner to the reactor and harvest liquor containing the biochemical product of interest is recovered. The biochemical product may comprise a primary or secondary metabolite of an immobilized organism, excess biomass generated by an immobilized organism containing for example a non-secretory product, an immobilized enzyme catalyzed reaction product or the like.

A particular advantage of the microsponge of the present invention is that it can be used in a mixed or motive system such as a fluidized bed reactor. As used herein, the term "motive reactor" refers to reactor systems in which relative motion between the microsponge and the fluid medium is provided in part by imparting motion to the microsponges themselves. Such reactor systems substantially enhance mass and energy transfer. A particularly preferred motive reactor system is described in co-pending U.S. Patent Application Serial No. 706,872 filed on February 28, 1985 in the names of Robert C. Dean, Jr., Peter V. Grela and Subhash B. Karkare.

To prepare a highly crosslinked collagen microsponge, a suitable collagen source first is milled to a small "fiber" size. Generally, the collagen is milled (e.g., using a Wiley mill) to obtain particles (fibers) with a maximum dimension on the order of about 200 microns. Preferably, the collagen source material is milled (i.e., dry ground) to yield fibers having a diameter on the order of 1 to 50 microns and a length no greater than about 200 microns. Proper milling of the collagen source material is important for obtaining microsponges of the desired structure.

The milled collagen then is formed into a collagen-based solution or dispersion, i.e., a soluble collagen dissolved in a solvent, or an insoluble collagen dispersed in a solvent by admixture with a suitable solvent, particularly acids such as dilute hydrochloric acid, dilute acetic acid or the like. In the present invention organic acids are particularly preferred, including acetic acid, lactic acid, proprionic acid, butyric acid and the like. Certain long chain fatty acids also could be used. The collagen is mixed into the liquid (solvent) using standard mixing equipment. Preferably, in the case of a collagen dispersion the mixing is accomplished with a high level of agitation using, for example, a Waring blender, so as to produce microfibers of the collagen. The mixture of collagen and solvent typically comprises between about 0.5% to about 1.5% by weight of the collagen. The mixture preferably exhibits a pH in the range of about 2.0 to about 4.0. A pH in the range of 1.0 to 2.0 may also be used as long as the temperature of the mixture is sufficiently reduced (e.g., about 4°C) to avoid denaturization of the collagen.

Next, the weighting additive is blended with the collagen-liquid mixture and the composite mixture is formed into small droplets and rapidly solidified by freezing at a temperature below about 0°C and preferably below about -30°C to form particles of the desired size. Any known technique for producing small particles can be employed in carrying out the present invention. Suitable techniques include, inter alia, pressure or air-shear spraying, emulsification techniques, droplet formation using Raleigh liquid jet instability techniques, extrusion techniques, droplet formation using gravity or centrifugal forces, electrostatic droplet formation, and droplet formation using inertial forces. For example, suitably sized particles have been prepared using inertial forces to form small droplets at the orifice of a vibrating needle. The droplets can be frozen by allowing them to fall into a cryogenic bath of liquid nitrogen. Obviously other chilling baths for freezing the droplets could be used, e.g., chilled ethanol. Also, larger sized particles formed by freezing possibly could be reduced to the desired particle size by such destructive techniques as grinding and the like. Still additional techniques such as various coating methodologies, could be used to form microsponges having a solid core of the weighting additive. In this case a shell of the collagen matrix would surround the weighted core. Those skilled in the art will recognize other techniques suitable for forming small particles of the types described above and the present invention is not intended to be limited to any specific technique.

The pore size and structure of the collagen microsponge is influenced by a variety of factors. For example, changes in the collagen concentration appear to affect pore size, with higher collagen concentrations tending to yield smaller pore dimensions. The pH of the mixture and the specific acid used in preparing the mixture also affect the pore size and structure of the resultant microsponge. For example, it has been found that too low a pH tends to significantly limit the pore dimensions of the microsponge while higher pHs cause a distinct collagen phase to separate from the original solution or dispersion thereby preventing the formation of a porous structure and when a finely divided weighting additive is used it tends to remain in the dispersed phase. The rate of freezing also appears to influence the structure of the microsponge and the structure also will vary with changes in collagen type.

Thereafter, the frozen composite is vacuum freeze-dried preferably using conventional equipment operating at a vacuum of more than about 50 millitorr and at a temperature in the range of about 22°C to -100°C. The combination of freezing and drying is referred to as lyophilization.

The freeze-dried collagen matrix composite then is treated so as to crosslink the collagen. The collagen can be crosslinked using either chemical crosslinking agents preferably selected from the group consisting of a carbodiiamide or N-hydroxy succinimide-derived active esters (succinimidyl active esters), by severe dehydration at an elevated temperature or by a combination of these treatments. The strength and biostability of the collagen matrix so-prepared is influenced by the degree of crosslinking introduced through such treatment. These crosslinking methods provide a collagen matrix that is surprisingly resistant to collagenase and other enzymatic degradation thereby making these materials particularly suitable for culturing organisms. Examples of carbodiiamides which can be used in the chemical treatment are cynamide and 1-ethyl-3-(3-dimethylaminopropyl)-carbodiiamide hydrochloride. Suitable bifunctional succinimidyl active esters include bifunctional N-hydroxy succinimide, 3,3'-dithio(sulfosuccinimidyl) proprionate and bis(sulfosuccinimidyl) suborate. When using such chemical crosslinking agents, the dry collagen matrix material is immersed in a solution of the crosslinking agent at about room temperature for a period of time of from about 2 to 96 hours. The solution of crosslinking agent may contain from about 0.1 to about 15% (weight per volume) of the crosslinking agent. Alternatively, the crosslinking agent could be added to the original solution or dispersion of the collagen source.

To crosslink the collagen matrix using severe dehydration, the microsponge is subjected to a vacuum of about 50 millitorr or more for a period of time of from about 2 to about 96 hours at a temperature in the range of about 50°C to about 200°C, e.g., about 100° to 110°C.

As noted above, the two treatments also can be used in combination and it is preferred to use a severe dehydration treatment followed by chemical treatment to crosslink the collagen matrix. Generally, however, in those cases where chemical treatment precedes the dehydration treatment the crosslinking agent should be added directly to the original collagen solution or dispersion prior to formulation of the matrix particles and lyophilization in order to facilitate subsequent vacuum dehydration treatment. Also as noted above the strength and biostability of the collagen matrix is influenced by the degree of crosslinking introduced through such treatment. These crosslinking methods provide a collagen matrix that is surprisingly resistant to collagenase and other enzymatic degradation thereby making these materials particularly suitable for culturing organisms. Whenever chemical treatment is used, the collagen matrix should be washed extensively prior to further use in order to remove any excess crosslinking agent. Further information concerning the procedure for preparing the highly crosslinked collagen can be obtained from U.S. Serial No. 593,733 filed on March 17, 1984 in the names of Frederick H. Silver, Richard A. Berg, David E. Birk, Kevin Weadock and Conrad Whyne and entitled "Biodegradable Matrix and Methods for Producing Same," the disclosure of which is incorporated herein by reference.

After thoroughly washing the crosslinked collagen matrix in ultra-pure water, the microsponges then may be sterilized using conventional sterilization techniques. A particular advantage of the collagen microsponges is that they are manufactured separate from the step of organism immobilization and as a result they can be properly sterilized prior to being inoculated or stored. Preferably, the microsponges are sterilized using gamma irradiation. Ethylene oxide also may be used as an alternative, as may additional sterilization procedures known to those skilled in the art, as long as the important characteristics of the microsponge are not compromised. Obviously, when sterilizing the microsponges using ethylene oxide the particles must be thoroughly ventilated in order to remove all traces of this sterilizing agent before subsequently using the microsponges for culturing organisms. It also has been discovered that the severe dehydration treatment for an extended time at an elevated temperature used to crosslink the collagen may satisfactorily sterilize the microsponge, thus obviating any additional treatment.

Preferably, the sterilized microsponges are aseptically packaged for delivery to the ultimate consumer. The user simply places the microsponges into a previously sterilized reactor, adds the proper nutrients and inoculum and initiates operation. In a preferred embodiment, the package actually comprises a disposable reactor vessel having the necessary connections for feeding a nutrient stream, for removing a harvest liquor and for ancillary operations, as needed, such as heat exchange, oxygenation and process control. For a fluidized bed reaction, the vessel also would contain a suitably designed distribution plate. Such a pre-packaged disposable reactor vessel may have a volume between about 0.1 liter and 10 liters. In this case, the user of the reactor simply integrates it into the process equipment consisting of pumps, valves, piping, heat and gas exchangers and various instrumentation and related probes and begins operation. Providing a disposable reactor, pre-packaged with the microsponges sterilized and ready for use, significantly simplifies start-up procedures for culturing organisms, particularly when changing from one culture to another.

Although not completely understood, it has been observed that variations in process parameters lead to important variations in the structure or configuration of the highly crosslinked collagen matrix itself. Figures 1 and 2, which are photomicrographs of the collagen matrix prepared using the techniques described above, illustrate these different structures. The photomicrographs of Figures 1 and 2 were obtained using scanning electron microscopy. Figure 1 illustrates a collagen matrix having substantially a wire-mesh structure. In this structure, the diameter of the fiber network typically is on the order of about 1 micron. This structure is particularly desirable for attachment-independent type organisms such as hybridomas. In this matrix, such organisms become trapped in and on the matrix structure. Figure 2 illustrates a leaf-type matrix structure. The leaves of this structure typically have a thickness on the order of about 1 micron. This structure is particularly suitable for attachment-dependent cells such as fibroblast cells. Currently, it is believed that the rate of chilling (freezing) the droplets in the bead making process influences the morphology of the collagen microsponge.

The following examples are intended to more fully illustrate the invention without acting as a limitation on its scope.

### Example 1

This example describes the preparation of microsponges of the highly crosslinked collagen matrix material.

Partially purified tendon collagen was milled to obtain fibers having a length of less than about 200 microns and a diameter of between about 5 and 50 microns using a Wiley Mill obtained from VWR Scientific. An amount of milled collagen material then was mixed with a solution of acetic acid using a Waring blender so as to produce a collagen-based dispersion having a pH of about 3.0 and about 1.0% (by weight) of collagen. Then, an inert weighting material, titanium, was added to the collagen-based dispersion as a fine powder having particle sizes within the range of about 5 to about 180 microns.

The composite mixture then was formed into solid particles by first producing small droplets of the composite mixture. Droplets were produced by flowing the composite mixture through a vibrating hollow needle having an internal diameter of about 1.3 millimeters vibrated at a frequency of about 90 Hz. The droplets fell into a cryogenic bath of liquid nitrogen and were rapidly frozen. The frozen droplets of the composite mixture then were vacuum dried using a Virtis Freezemobile Lyophilizer Model 6 at a vacuum of about 10 millitorr for about 48 hours.

After such lyophilization, the dried microsponges were subjected to a severe dehydration (dehydrothermal treatment) at a temperature of about 100 °C under a vacuum of about 10 millitorr for about 72 hours using a VWR Scientific drying oven. The microsponges then were treated with a 1.0% (by weight) solution of cyanamide as a chemical crosslinking agent at a pH of about 5.5 for about 24 hours at about 20°C.

The highly crosslinked microsponges then were thoroughly washed for about 24 hours using ultra-pure water, were dried and then sterilized by gamma irradiation. The microsponges had particle sizes within the range of about 200 to 800 microns, a void fraction on the order of about 77%, pore sizes on the order of about 20 microns, and a specific gravity on the order of about 1.1. The microsponges had a wire mesh micro-structure.

### Example 2

The microsponges of Example 1 can be used to support the growth of hybridoma cells. In particular, about 300 ml of the microsponges can be contained in a 600 ml reactor vessel. The microsponges can be inoculated with the hybridoma cells and cultured using a suitable nutrient medium. The reactor can be operated at a solids concentration of about 25-40%, while the content of the reactor is vigorously agitated. A nutrient medium such as Delbecko Modified Eagle medium with 10% fetal calf serum can be passed into the reactor in a continuous manner and a product stream containing the monoclonal antibodies can be recovered at a substantially equivalent flow rate.

It will be obvious to one of ordinary skill that numerous modifications may be made without departing from the true spirit and scope of the invention which is to be limited only by the appended claims.

## Claims

1. A microsponge for immobilizing bioactive materials in motive bioreactor systems, being characterized in that the microsponge comprises a porous, biostable matrix of a biocompatible polymer containing an inert weighting material which weights the microsponge; the matrix having an open to the surface pore structure with an average pore size in the range of from about 1 to about 150 microns, the pores of the matrix occupying from about 70 to about 98% by volume of the microsponge, and in that the microsponge has an average particle size of from about 100 to 1000 microns and a specific gravity of above about 1.05.

2. A microsponge according to claim 1 having immobilized therein bioactive material selected from the group consisting of enzymes, microorganisms, dead cells and living cells.

3. A microsponge according to claim 1 wherein said biostable, biocompatible polymer is selected from collagen, cellulose, dextran, dextrin, polyamides, polyesters, starch, agarose, carrageenin, polyurethanes, polyvinyl alcohols, polyacrylates, polymethacrylates and polyacrylamides.

4. A microsponge according to claim 1 wherein the inert weighting material is selected from metals, metal alloys, metal oxides and ceramics.

5. A microsponge according to claim 4 wherein the weighting materials has a specific gravity of above about 4.0 and the microsponge has a specific gravity of above about 1.3.

6. A microsponge according to claim 5 wherein the inert weighting material is dispersed throughout the porous matrix as finely divided powder.

7. A microsponge according to claim 5 wherein the weighting material is centrally disposed as a solid core about which the porous matrix is formed.

8. A microsponge according to claim 5 wherein the inert weighting material is selected from chromium, tungsten, cobalt, molybdenum, titanium, nickel and alloys.

9. A microsponge according to claim 8 wherein the weighting material is titanium and the microsponge has hybridoma cells immobilized therein.

10. A microsponge according to claim 1 wherein the bioactive biocompatible polymer is highly crosslinked collagen.

11. A microsponge according to claim 10 wherein the highly crosslinked collagen matrix is prepared by the steps of
(a) milling a source of collagen selected from Type I, II and III collagen,
(b) admixing the milled collagen with an acidic liquid medium,
(c) lyophilizing the collagen-liquid medium mixture into a dry matrix, and
(d) crosslinking the collagen in the dry matrix by a treatment selected from
(i) contacting the collagen with a crosslinking agent selected from the group consisting of carbodiimides and bifunctional suiccinimidyl active esters,
(ii) subjecting the solid droplet to elevated temperatures under a vacuum, and
(iii) a combination thereof.

12. A microsponge according to claim 11 wherein the inert weighting material is added into the mixture of collagen and acidic liquid medium as a finely divided powder prior to lyophilization.

13. The microsponge of claim 12 wherein the collagen is Type 1, tendon collagen.

14. A bioreactor system comprising a plurality of weighted microsponges according to claim 1 sterilized and aseptically sealed in a reactor vessel.

15. A bioreactor system according to claim 14 wherein the reactor has a volume between about 0.1 to 10 litres.

16. A bioreactor system according to claim 15 wherein the reactor is a fluidized bed reactor, having a fluid distribution plate.

17. A process for performing a bioreaction comprising immobilizing a bioactive material in the microsponges of claim 1; containing the microsponges having the immobilized bioactive material in a suitable reactor vessel; passing a liquid reagent stream into the reactor in direct contact with the microsponges; agitating the mixture of the microsponges and the reagent stream and recovering the biochemical reaction products from the reactor.

18. A process according to claim 17 wherein organisms are immobilized in the microsponges, the microsponges are incubated to promote growth and colonization of the microsponges by the organisms, the reagent comprises nutrient media for promoting the growth and metabolism of the organisms, and wherein the product comprises metabolites of the organisms.

19. A process of claim 17 wherein organisms are immobilized on the microsponges and the recovered product comprises free organisms which have escaped from the microsponges.

20. A process according to claim 18 wherein the organisms comprise hybridomas and the product comprises monoclonal antibodies.

21. A process according to claim 20 wherein the reactor vessel comprises a fluidized bed reactor.

22. A process according to claim 18 wherein the organisms comprise mammalian cells and the products comprise mammalian cell products.

23. A process according to claim 18 wherein the organisms are genetically engineered microbial cells and the product comprises secreted protein products.

24. A process according to claim 19 wherein the organisms are genetically engineered microbial cells and the product comprises the organisms containing a non-secreted protein product.

25. A method for the continuous in vitro culture of organisms for the production of biochemical products comprising the steps of:
(a) providing a highly crosslinked, collagen microsponge prepared by
(i) milling a source of collagen selected from the group consisting of Types I, II and III collagen,
(ii) admixing the milled collagen with an acidic liquid medium,
(iii) lyophilizing the collagen-liquid medium mixture into a dry sponge matrix, and
(iv) crosslinking the collagen in the sponge matrix by a treatment selected from:
(1) contacting the collagen with a crosslinking agent selected from the group consisting of carbodiimides and bifunctional succinimidyl active esters,
(2) subjecting the dry sponge matrix to elevated temperatures under a vacuum, and
(3) a combination thereof;
(b) inoculating the highly crosslinked, collagen microsponge of step (a) with a culture of the organisms;
(c) passing a nutrient media in direct contact with the inoculated, highly crosslinked collagen microsponge; and
(d) recovering the biochemical products with the nutrient media effluent.

26. A method according to 25 wherein the organisms are selected from bacteria, fungi, viruses, algae, yeasts, animal cells and plant cells.

27. A method of claim 25 wherein the organisms are mammalian cells and the biochemical products comprise mammalian cell products.

28. A method according to claim 25 wherein the organisms are hybridomas and the biochemical products comprise monoclonal antibodies.

29. A method according to claim 25 wherein the organisms are genetically engineered and the biochemical products comprise the organisms containing a non-secreted protein product.

30. A method according to claim 25 wherein the organisms are genetically engineered and the biochemical products comprise secreted protein products.

31. A method according to claim 25 wherein the highly crosslinked, collagen microsponge is formed into a shape selected from beads, flakes, discs, fibres, films and sheets.

32. A method according to claim 25 wherein said highly crosslinked, collagen microsponge is provided as a coating on a support.

33. A method according to claim 25 wherein the highly crosslinked, collagen microsponge has an open to the surface pore structure with an average pore size in the range of from about 1 to about 150 microns and the pores of the microsponge occupy from about 70 to about 98% by volume of the matrix.

34. A method according to claim 25 wherein the highly crosslinked, collagen microsponge is in the shape of a bead with an average particle size of from about 100 to 1000 microns.

35. A method of claim 25 wherein the nutrient media is passed in direct contact with the inoculated, highly crosslinked collagen microsponge in a reactor selected fixed bed reactors, stirred tank reactors, fluidized bed reactors and moving bed reactors.

36. A method according to claim 25 wherein the highly crosslinked collagen in the microsponge has a molecular weight of from about 1 X 10⁶ to about 50 X 10⁶.

37. A method according to claim 25 wherein the highly crosslinked collagen has a molecular weight between crosslinks of from about 1,000 to 100,000 via covalent bonding.

## Patentansprüche

1. Ein Mikroschwamm zur Immobilisierung/Festlegung bioaktiven Materials in bewegten Bioreaktor-Systemen,
dadurch gekennzeichnet,
daß der Mikroschwamm besteht aus einer porösen, biostabilen (biologisch stabilen) Matrix aus biokompatiblem (biologisch verträglichem) Polymer, welches ein inertes Beschwerungsmaterial zur Beschwerung des Mikroschwamms enthält; die Matrix hat eine zur Oberfläche offene Porenstruktur mit einer durchschnittlichen Porengröße von ungefähr 1 bis ca. 150 Mikrons, die Poren der Matrix nehmen ungefähr 70 Vol-% bis 98 Vol-% des Volumens des Mikroschwammes ein (ca. 70 bis 98 Vol-% Porenvolumen), und daß der Mikroschwamm eine durchschnittliche Partikelgröße von ungefähr 100 bis 1000 Mikrons und ein spezifisches Gewicht von ungefähr 1,05 hat.

2. Ein Mikroschwamm nach Anspruch 1 mit darin immobilisiertem bioaktivem Material,
welches ausgewählt ist aus der Gruppe bestehend aus Enzymen, Mikroorganismen, toten Zellen und lebenden Zellen.

3. Ein Mikroschwamm nach Anspruch 1,
wobei das biostabile, biokompatible Polymer ausgewählt ist aus den Stoffgruppen Collagenen, Zellulose, Dextran, Dextrin, Polyamiden, Polyestern, Stärke, Agarose, Carragenin, Polyurethan, Polyvinyl-Alkohol, Polyacrylat, Polymethacrylat und Polyacrylamid.

4. Ein Mikroschwamm nach Anspruch 1,
wobei das inerte Beschwerungsmaterial ausgewählt ist aus Metallen, Metallegierungen, Metalloxyden und Keramiken.

5. Ein Mikroschwamm nach Anspruch 4,
wobei das Beschwerungsmaterial ein spezifisches Gewicht von ungefähr 4.0 und der Mikroschwamm ein spezifisches Gewicht von ungewähr 1,3 hat.

6. Ein Mikroschwamm nach Anspruch 5,
wobei das inerte Beschwerungsmaterial dispergiert bzw. fein verteilt ist über die poröse Matrix als fein verteiltes Pulver.

7. Ein Mikroschwamm nach Anspruch 5,
wobei das Beschwerungsmaterial zentral angeordnet ist als ein fester Kern, um den herum die poröse Matrix gebildet ist.

8. Ein Mikroschwamm nach Anspruch 5,
wobei das inerte Beschwerungsmaterial/Gewichtsmaterial ausgewählt ist von Chrom, Wolfram, Kobald, Molybdän, Titan, Nickel und deren Legierungen.

9. Ein Mikroschwamm nach Anspruch 5,
wobei das Gewichtsmaterial Titan ist und Hybridoma-Zellen in den Mikro-Schwamm immobilisiert sind.

10. Mikroschwamm nach Anspruch 1,
wobei das bioaktive, biokompatible Polymer in hohem Maße über Kreuz verbundenes Collagen ist.

11. Mikroschwamm nach Anspruch 10,
wobei die kreuzweise verbundene Collagen-Matrix gebildet ist durch die Verfahrensstufen:
(a) Mahlen eines Collagenmaterials ausgewählt von den Typen I, II und III Collagen,
(b) Vermischen des gemahlenenen Collagens mit einem sauren flüssigen Medium,
(c) Gefriertrocknen der flüssigen Collagenmischung in eine trockene Matrix und
(d) kreuzweises Verbinden des Collagens in der trockenen Matrix durch eine Behandlung ausgewählt von
(I) Kontaktieren/Inberührungbringen des Collagens mit einem querverbindenden Mittel, ausgewählt von der Gruppe bestehend aus Carbodiimid und bifunktionalem Suiccinimidyl aktiven Estern,
(II) Aussetzen bzw. Unterwerfen der festen Tröpfchen erhöhten Temperaturen unter Vakuum und
(III) einer Kombination hiervon.

12. Mikroschwamm nach Anspruch 11,
wobei das inerte Gewichtsmaterial als fein verteiltes Pulver vor der Gefriertrocknung in die Mischung von Collagen und saurem, flüssigem Mittel zugesetzt wird.

13. Der Mikroschwamm nach Anspruch 12,
wobei das Collagen vom Typ 1 ist, Tendon Collagen.

14. Ein Bioreaktor-System bestehend aus einer Vielzahl beschwerter
Mikro-Schwämme gemäß Anspruch 1, die in einem Reaktorbehälter sterilisiert und aseptisch versiegelt sind.

15. Ein Bioreaktor-System gemäß Anspruch 14,
wobei der Reaktor ein Volumen zwischen 0,1 bis 10 Litern hat.

16. Ein Bioreakter-System gemäß Anspruch 15,
wobei der Reaktor ein Fluid-Bett-Reaktor mit einer Fluidverteilungsplatte ist.

17. Ein Verfahren zur Ausführung einer Bioreaktion, umfassend eine Immobilisierung eines bioaktiven Materials in Mikroschwämmen gemäß Anspruch 1; Einbringen der Mikroschwämme mit dem immobilisierten bioaktiven Material in einen passenden Reaktorbehälter; Durchleiten eines flüssigen Reaktionsmittel-Stromes in und durch den Reaktor in direktem Kontakt mit den Mikroschwämmen, Rühren der Mischung aus Mikroschwämmen und strömendem Reaktionsmittel, Gewinnen des biochemischen Reaktionsproduktes aus dem Reaktor.

18. Ein Verfahren gemäß Anspruch 17,
wobei Organismen in den Mikroschwämmen immobilisiert sind, wobei die Mikroschwämme vorbereitet/ausgebrütet sind zur Förderung des Wachstums und zur Colonisierung der Mikroschwämme durch die Organismen, wobei das Reaktionsmittel Nährmittel zur Förderung des Wachstums und der Metabolisierung der Organismen enthält, und wobei das Produkt Metabole (Stoffwechselprodukte) der Organismen enthält.

19. Ein Verfahren nach Anspruch 17,
wobei die Organismen an bzw. auf den Mikroschwämmen immobilisiert sind und das gewonnene Produkt freie Organismen enthält, die von den Mikroschwämmen entwichen sind.

20. Ein Verfahren nach Anspruch 18,
wobei die Organismen aufweisen bzw. bestehen aus Hybridomas und das Produkt monoclonale Antikörper aufweist.

21. Verfahren nach Anspruch 20,
wobei der Reaktorbehälter einen Fluid-Bett-Reaktor aufweist.

22. Verfahren nach Anspruch 18,
wobei die Organismen Säugetierzellen und das Produkt Säugetierzellprodukte umfassen.

23. Ein Verfahren nach Anspruch 18,
wobei die Organismen durch Gentechnik hergestellte Mikro-Zellen sind und das Produkt ausgeschiedene Proteinprodukte umfaßt.

24. Ein Verfahren nach Anspruch 19,
wobei die Organismen durch Gentechnik hergestellte Mikroben-Zellen sind und das Produkt Organismen umfaßt, welche ein nicht ausgeschiedenes Protein-Produkt enthält.

25. Ein Verfahren für die kontinuierliche In-Vitro-Kultur von Organismen für die Herstellung biochemischer Produkte, umfassend die Schritte:
(a) Bereitstellung eines hochgradig querverbundenen Collagen Mikroschwammes, der hergestellt wurde durch
(I) Mahlen einer Quelle von Collagen, welches ausgewählt ist von der Gruppe bestehend aus den Typen I, II und III Collagenen,
(II) Mischen des gemahlenen Collagens mit einem sauren, flüssigen Medium,
(III) Gefriertrocknen der Collagen-Flüssigkeitsmischung zu einer trockenen schwammigen Matrix und
(IV) Querverbindung des Collagens in der Schwamm-Matrix durch eine Behandlung ausgewählt aus
(1) Inkontaktbringen des Collagens mit einem Querverbindungselement, ausgewählt aus der Gruppe bestehend aus Carbodiimiden und bifunktionalen Succinimidyl aktiven Estern,
(2) Unterwerfen der trockenen schwammigen Matrix erhöhten Temperaturen und einem Vakuum, und
(3) einer Kombination davon;
(b) Impfen des hoch querverbundenen Collagen-Mikroschwammes der Stufe (a) mit einer Kultur des Mikro-Organismus;
(c) Durchleiten einer Nährlösung in direktem Kontakt mit dem geimpften, hochgradig querverbundenen Collagen-Mikroschwammes und
(d) Gewinnung des biochemischen Produktes aus der abfließenden Nährlösung.

26. Ein Verfahren nach Anspruch 25,
wobei die Organismen ausgewählt sind aus Bakterien, Pilzen, Viren, Algen, Hefen, tierischen Zellen und pflanzlichen Zellen.

27. Ein Verfahren nach Anspruch 25,
wobei die Organismen Säugetierezellen sind und die biochemischen Produkte Säugetierzell-Produkte umfassen.

28. Ein Verfahren nach Anspruch 25,
wobei die Organismen Hybridomas sind und die biochemischen Produkte monoclonale Antikörper umfassen.

29. Ein Verfahren gemäß Anspruch 25,
wobei die Organismen gentechnisch hergestellt sind und die biochemischen Produkte Organismen umfassen, die ein nicht ausgeschiedenes Protein-Produkt enthalten.

30. Ein Verfahren gemäß Anspruch 25,
wobei die Organismen gentechnisch behandelt/hergestellt sind und die biochemischen Produkte ausgeschiedene (sekretierte) Protein-Produkte umfassen.

31. Ein Verfahren gemäß Anspruch 25,
wobei der hochgradig querverbundenen Collagen-Mikroschwamm gebildet ist in einer Form ausgewählt aus Perlen, Flocken, Scheiben, Basen, Filmen und Bogen.

32. Ein Verfahren gemäß Anspruch 25,
wobei der hochgradig querverbundene Collagen-Mikroschwamm vorgesehen ist als ein Überzug auf einem Träger.

33. Ein Verfahren gemäß Anspruch 25,
wobei der hochgradig querverbundene Collagen-Mikroschwamm eine zu den Oberflächen offene Porenstruktur hat mit einer durchschnittlichen Porengröße im Bereich von ungefähr 1 bis ungefähr 150 Mikrons und die Poren des Mikroschwammes ungefähr 70 bis ungefähr 98 Vol.-% der Matrix einnehmen.

34. Ein Verfahren gemäß Anspruch 25,
wobei der hochgradig querverbundene Collagen-Mikroschwamm vorliegt in der Gestalt von Perlen mit einer durchschnittlichen Partikelgröße von ungefähr 100 bis 1000 Mikron.

35. Ein Verfahren nach Anspruch 25,
wobei das Nährmedium durchgeleitet wird in direktem Kontakt mit den ungeimpften, hochgradig querverbundenem Collagen-Mikroschwamm in einem Reaktor ausgewählt aus Fest-Bett-Reaktoren, Rührtank-Reaktoren, Fluid-Bett-Reaktoren und Bewegt-Bett-Reaktoren.

36. Ein Verfahren gemäß Anspruch 25,
wobei die hochgradig querverbundene Collagen in dem Mikroschwamm ein Molekulargewicht von ungefähr 1 x 10⁶ bis ungefähr 50 x 10⁶ hat.

37. Ein Verfahren gemäß Anspruch 25,
wobei das hochgradig querverbundene Collagen zwischen den Querverbindungen ein Molekulargewicht von ungefähr 1000 bis 100000 Kovalenzbindungen (Elektronenpaarbindungen) aufweist.

## Revendications

1. Une microéponge permettant d'immobiliser des matières bio-actives dans les systèmes de bio-réacteurs ouverts, caractérisée en ce que la microéponge comprend une matrice bio-stable poreuse constituée d'un polymère bio-compatible, comprenant un matériau alourdissant inerte qui alourdit la micro-éponge; la matrice présentant une ouverture au niveau de la surface poreuse avec une dimension moyenne des pores comprise dans un intervalle allant d'environ 1 à environ 150 microns, les pores de la matrice occupant environ 70 à environ 98% en volume de la micro-éponge, et en ce que la micro-éponge a une taille moyenne de particules d'environ 100 à 1000 microns et une densité spécifique d'environ 1,05.

2. Une microéponge selon la revendication 1, dans laquelle sont immobilisées les matières bio-actives sélectionnées dans le groupe consistant en : enzymes, micro-organismes, cellules mortes et cellules vivantes.

3. Une microéponge selon la revendication 1, caractérisée en ce que le polymère bio-compatible bio-stable est sélectionné parmi collagène, cellulose, dextran, dextrine, polyamides, polyesters, amidon, agarose, carragénine, polyuréthanes, alcools polyvinyliques, polyacrylates, polyméthacrylates et polyacrylamides.

4. Une microéponge selon la revendication 1, caractérisée en ce que le matériau alourdissant inerte est sélectionné parmi des métaux, des alliages de métaux, des oxydes métalliques et des céramiques.

5. Une microéponge selon la revendication 4, caracté risée en ce que le matériau alourdissant a une densité spécifique supérieure à environ 4,0 et la microéponge a une densité spécifique supérieure à environ 1,3.

6. Une microéponge selon la revendication 5, caractè risée en ce que le matériau alourdissant inerte est dispersé de façon homogène dans la matrice poreuse sous la forme d'une poudre finement divisée.

7. Une microéponge selon la revendication 5, caractérisée en ce que le matériau alourdissant est disposé au centre sous la forme d'un noyau solide autour duquel la matrice poreuse est formée.

8. Une microéponge selon la revendication 5, caractérisée en ce que le matériau alourdissant inerte est sélectionné parmi chrome, tungstène, cobalt, molybdène, titane, nickel et les alliages.

9. Une microéponge selon la revendication 8, caractérisée en ce que le matériau alourdissant est du titane et des cellules d'hybridomes sont immobilisées dans la microéponge.

10. Une microéponge selon la revendication 1, caractérisée en ce que le polymère bio-compatible bio-actif correspond à du collagène hautement réticulé.

11. Une microéponge selon la revendication 10, caractérisée en ce que la matrice de collagène hautement réticulé est préparée par les étapes suivantes :
(a) broyage d'une source de collagène sélectionnée parmi les collagènes du type 1, 2 et 3,
(b) mélange du collagène broyé avec un milieu liquide acide,
(c) liophilisation du mélange milieu liquide-collagène dans une matrice sèche, et
(d) réticulation du collagène dans la matrice sèche par un traitement sélectionné parmi
(i) la mise en contact du collagène avec un agent de réticulation sélectionné dans le groupe consistant en carbodiimides et esters actifs bifonctionnels de suiccinimidyl,
(ii) l'exposition des particules solides à des températures élevées sous vide et,
(iii) une combinaison de ces traitements.

12. Une microéponge selon la revendication 11, caractérisée en ce que le matériau alourdissant inerte est ajouté au mélange de collagène et de milieu acide sous la forme d'une poudre finement divisée avant l'étape de liophilisation.

13. La microéponge selon la revendication 12, caractérisée en ce que le collagène est un collagène de tendon de type 1.

14. Un système de bio-réacteur comprenant une pluralité d'éponges chargées, selon la revendication 1, stérilisées et scellées de façon aseptique dans le récipient du réacteur.

15. Un système de bio-réacteur selon la revendication 14, caractérisé en ce que le réacteur a un volume compris entre environ 0,1 et 10 litres.

16. Un système de bio-réacteur selon la revendication 15, caractérisé en ce que le réacteur est un réacteur à lit fluidisé comportant une plaque de distribution du fluide.

17. Un procédé permettant de réaliser une bio-réaction comprenant l'immobilisation d'une matière bio-réactive dans des microéponges selon la revendication 1; le chargement des microéponges comportant la matière du réactif immobilisé dans un récipient de réacteur approprié; le passage d'un courant de réactif dans le réacteur au contact direct avec les microéponges; l'agitation du mélange des microéponges et du courant de réactif et la récupération des produits de réaction biochimique à partir du réacteur.

18. Un procédé selon la revendication 17, caractérisé en ce que des microorganismes sont immobilisés dans les microéponges, les microéponges sont incubées pour favoriser la croissance et la colonisation des microéponges par les microorganismes, le réactif comprend un milieu nutritif permettant de favoriser la croissance et le métabolisme des microorganismes, et en ce que le produit comprend les métabolites des microorganismes.

19. Un procédé selon la revendication 17, caractérisé en ce que des microorganismes sont immobilisés sur les microéponges, et le produit récupéré comprend les microorganismes libres qui se sont échappés des microéponges.

20. Un procédé selon la revendication 18, caractérisé en ce que les microorganismes comprennent des hybrodomes et le produit comprend des anticorps monoclonaux.

21. Un procédé selon la revendication 20 caractérisé en ce que le récipient du réacteur comprend un réacteur à lit fluidisé.

22. Un procédé selon la revendication 18, caractérisé en ce que les microorganismes comprennent des cellules de mammifères et les produits comprennent des produits de cellules de mammifères.

23. Un procédé selon la revendication 18, caractérisé en ce que les microorganismes sont des cellules microbiennes génétiquement modifiées et le produit comprend des produits protéiques sécrétés.

24. Un procédé selon la revendication 19, caractérisé en ce que les microorganismes sont des cellules microbiennes génétiquement modifiées et le produit comprend des organismes contenant un produit protéique non-sécrété.

25. Un procédé permettant la culture in vitro en continu d'organismes pour la production de produits biochimiques comprenant les étapes suivantes :
(a) utilisation d'une microéponge à base de collagène hautement réticulé préparé par :
(i) broyage d'une source de collagène sélectionnée dans le groupe consistant en collagène de type 1, 2 et 3,
(ii) mélange du collagène broyé avec un milieu liquide acide,
(iii) liophilisation du mélange milieu liquide-collagène dans une matrice d'éponge sèche, et
(iv) réticulation du collagène dans la matrice d'éponge par un traitement sélectionné parmi :
(1) la mise en contact du collagène avec un agent de réticulation sélectionné dans le groupe consistant en carbodiimides et les esters actifs bifonctionnels de succinimidyl,
(2) l'exposition de la matrice d'éponge sèche à des températures élevées sous vide, et
(3) une combinaison de ces traitements;
(b) inoculation de la microéponge à base de collagène hautement réticulée de l'étape (a) avec une culture de microorganismes;
(c) passage d'un milieu nutritif au contact direct de la microéponge inoculée à base de collagène hautement réticulé; et
(d) récupération des produits biochimiques avec l'effluent de milieu nutritif.

26. Un procédé selon la revendication 25, caractérisé en ce que les microorganismes sont sélectionnés parmi bactéries, champignons, virus, algues, levures, cellules animales et cellules végétales.

27. Un procédé selon la revendication 25, caractérisé en ce que les microorganismes sont des cellules de mammifères et les produits chimiques comprennent des produits de cellules de mammifères.

28. Un procédé selon la revendication 25, caractérisé en ce que les microorganismes sont des hybridomes et les produits biochimiques comprennent des anticorps monoclonaux.

29. Un procédé selon la revendication 25, caractérisé en ce que les microorganismes sont modifiés génétiquement et les produits biochimiques comprennent les organismes contenant un produit protéique non-sécrété.

30. Un procédé selon la revendication 25, caractérisé en ce que les organismes sont modifiés génétiquement et les produits biochimiques comprennent des produits protéiques sécrétés.

31. Un procédé selon la revendication 25, caractérisé en ce que la microéponge à base de collagène hautement réticulé est formée sous une forme sélectionnée parmi billes, flocons, disques, fibres, films et feuilles.

32. Un procédé selon la revendication 25, caractérisé en ce que ladite microéponge à base de collagène hautement réticulé est préparée sous la forme d'un revêtement sur un support.

33. Un procédé selon la revendication 25, caractérisé en ce que la microéponge à base de collagène hautement réticulé présente une ouverture au niveau de la surface poreuse avec une dimension moyenne des pores comprise dans un intervalle allant d'environ 1 à environ 150 microns, et les pores de la microéponge occupent environ de 70 à environ 98% en volume de la matrice.

34. Un procédé selon la revendication 25, caractérisé en ce que la microéponge à base de collagène hautement véhiculé est sous forme de billes ayant une dimension particulaire moyenne d'environ 100 à 1000 microns.

35. Un procédé selon la revendication 25, caractérisé en ce que le milieu nutritif passe au contact direct de la microéponge inoculée à base de collagène hautement réticulé dans un réacteur sélectionné parmi des réacteurs à lit fixe, des réacteurs agités, des réacteurs à lit fluidisé et des réacteurs à lit mobile.

36. Un procédé selon la revendication 25, caractérisé en ce que le collagène hautement réticulé présent dans la microéponge a un poids moléculaire compris entre environ 1.10⁶ et environ 50.10⁶.

37. Un procédé selon la revendication 25, caractérisé en ce que le collagène hautement réticulé a un poids moléculaire entre les liaisons de réticulation d'environ 1000 à environ 100000 par liaison covalente.
